**Europäisches Patentamt**

**European Patent Office**

**Office européen des brevets**

(19)

(11) Veröffentlichungsnummer : **0 043 088**
**B1**

# (12) EUROPÄISCHE PATENTSCHRIFT

(45) Veröffentlichungstag der Patentschrift :
**18.01.84**

(51) Int. Cl.³ : **C 07 C 69/738, C 07 C 67/313**

(21) Anmeldenummer : **81104851.1**

(22) Anmeldetag : **23.06.81**

(54) **Verfahren zur Herstellung von Acetessigsäurealkylestern.**

(30) Priorität : **28.06.80 DE 3024535**

(43) Veröffentlichungstag der Anmeldung :
**06.01.82 Patentblatt 82/01**

(45) Bekanntmachung des Hinweises auf die Patenterteilung : **18.01.84 Patentblatt 84/03**

(84) Benannte Vertragsstaaten :
**BE CH DE FR GB IT LI NL**

(56) Entgegenhaltungen :
**US-A- 2 900 311**

(73) Patentinhaber : **HOECHST AKTIENGESELLSCHAFT**
**Postfach 80 03 20**
**D-6230 Frankfurt am Main 80 (DE)**

(72) Erfinder : **Günther, Klaus, Dr.**
**Waldstrasse 5**
**D-6239 Eppstein/Taunus (DE)**
Erfinder : **Wendt, Heinz, Dr.**
**Kronberger Weg 20**
**D-6231 Sulzbach (DE)**

Verfahren zur Herstellung von Acetessigsäurealkylestern

Die technische Herstellung von Acetessigsäurealkylestern erfolgt heute i. allg. durch Umsetzung von Diketen mit dem entsprechenden wasserfreien aliphatischen Alkohol. Bei dieser Reaktion erhält man ein Rohprodukt, das außer dem gewünschten Acetessigsäurealkylester eine ganze Reihe von Niedrigsiedern, Mittelsiedern und Hochsiedern enthält. Als Niedrigsieder sind in dem Rohester vor allem überschüssiger Alkohol, Aceton und Alkylacetat enthalten. Als Hochsieder liegen i. allg. Dehydracetsäure und ein thermisch wenig beständiger Rückstand vor, der sich aus Diketen gebildet hat. An Mittelsiedern, die im Bereich des entsprechenden Acetessigsäurealkylesters sieden, treten vornehmlich 3-Acetoxycrotonsäurealkylester und zwar in der cis- und der trans-Form und 3-Acetoxyvinylessigsäurealkylester auf. Der reine Acetessigsäurealkylester wird aus diesem komplexen Gemisch durch fraktionierte Destillation gewonnen.

Während sich die Niedrigsieder sowie die Hochsieder dabei unschwer abtrennen lassen, ist dies bei den Mittelsiedern 3-Acetoxycrotonsäurealkylester und 3-Acetoxyvinylessigsäurealkylester — im folgenden meist als AC-Ester bzw. AV-Ester bezeichnet — nicht ohne weiteres möglich. Eine Abtrennung dieser Komponenten durch fraktionierte Destillation ist aufwendig und gelingt nur mit einem hohem Rücklaufverhältnis.

Eine einfachere Möglichkeit wäre theoretisch eine spezifische chemische Umwandlung dieser störenden Substanzen in andere, leichter destillativ abtrennbare Verbindungen. Jedoch war dabei zu berücksichtigen, daß der als Hauptkomponente des Gemischs vorliegende Acetessigsäurealkylester drei reaktive Gruppen enthält — eine Carbonylgruppe, eine Carbalkoxygruppe und eine aktive Methylengruppe — und somit sehr reaktionsfähig ist. Eine chemische Umwandlung der störenden Komponenten AC-Ester und AV-Ester ohne gleichzeitige unerwünschte Reaktion des Acetessigsäurealkylesters erschien daher nicht möglich.

Es wurde nun überraschenderweise gefunden, daß bei Einwirkung eines Alkohols auf AC- und/oder AV-Ester eine glatte Umwandlung dieser Verbindungen zu Acetessigsäurealkylester stattfindet, ohne daß dieser selbst mit dem Alkohol reagiert.

Das erfindungsgemäße Verfahren zur Herstellung von Acetessigsäurealkylester ist dadurch gekennzeichnet, daß man 3-Acetoxycrotonsäurealkylester oder 3-Acetoxyvinylessigsäurealkylester oder deren Gemisch bei einer Temperatur von 50-250 °C mit einem aliphatischen Alkohol, der 1 bis 5 Kohlenstoffatome enthält, in der Flüssigphase umsetzt, wobei der Alkohol in mindestens äquimolarer Menge eingesetzt wird.

Vorzugsweise haben die Alkylgruppen im 3-Acetoxycrotonsäurealkylester und im 3-Acetoxyvinylessigsäurealkylester 1 bis 5 C-Atome und können gesättigt oder ungesättigt, geradkettig oder verzweigt sein.

Bei der Reaktion des AC-Esters bzw. des AV-Esters mit dem Alkohol ensteht neben Acetessigsäurealkylester der Essigsäureester des eingesetzten Alkohols, d. h. das bereits oben als Niedrigsieder erwähnte Alkylacetat.

Man kann AC-Ester oder AV-Ester einzeln oder gemischt einsetzen.

Die beiden Ester kann man einzeln oder als Mischung aus dem Rohprodukt erhalten, das bei der Herstellung von Acetessigsäurealkylestern aus Diketen und einem Alkohol entsteht, indem man zunächst die Niedrig- und die Hochsieder abtrennt und dann durch fraktionierte Destillation AC-Ester, AV-Ester und Acetessigsäurealkylester isoliert. Man erhält auf diese Weise AC-Ester oder deren Gemisch, und zwar frei von Acetessigsäurealkylester.

Vorzugsweise verzichtet man jedoch auf diese relativ aufwendige vollständige Abtrennung des Acetessigsäurealkylesters und geht wie folgt vor : Man trennt wieder zunächst die Niedrig- und die Hochsieder aus dem genannten Rohprodukt destillativ ab, dann wird die den Acetessigsäurealkylester, den AC-Ester sowie den AV-Ester enthaltende Hauptfraktion in einer zweiten Destillation in reinen Acetessigsäurealkylester als Kopfprodukt und in ein Gemisch Acetessigsäurealkylester/AC-Ester/AV-Ester als Sumpfprodukt aufgetrennt. Dieses Gemisch wird dann erfindungsgemäß mit einem aliphatischen Alkohol bei 50-250 °C, vorzugsweise bei 140-180 °C umgesetzt, und zwar unter einem Druck, der dem Dampfdruck der Mischung entspricht. Das entstehende Reaktionsgemisch wird destillativ zu Acetessigsäurealkylester aufgearbeitet.

Man kann aber auch — obwohl dies in der Regel weniger günstig ist — die erwähnte, nach Abtrennung der Niedrig- und Hochsieder erhaltene Hauptfraktion gleich erfindungsgemäß mit einem aliphatischen Alkohol umsetzen und destilliert erst anschließend reinen Acetessigsäurealkylester als Kopfprodukt ab und erhält ein Sumpfprodukt, das den restlichen Acetessigsäurealkylester und den unumgesetzten AC- und AV-Ester enthält.

Entsprechend geht man natürlich mit reinem (dh. keinen Acetessigsäurealkylester enthaltendem) AC-Ester bzw. AV-Ester bzw. deren Gemisch vor.

Zweckmäßigerweise wird man beim Einsatz eines wie erwähnt erhaltenen AC/Ester/AV-Ester/Acetessigsäurealkylester-Gemisches den Alkohol verwenden, der bei der Herstellung des Acetessigsäurealkylesters aus Diketen eingesetzt wurde. Bei der Herstellung von Acetessigsäuremethylester ist dies Methanol, bei Acetessigsäureethylester Ethanol usw. Die umzusetzenden Komponenten AC-Ester und AV-Ester haben dabei eine Alkoxygruppe, die dem bei der Diketen-Reaktion eingesetzten Alkohol entspricht. Acetessigsäuremethylester enthält somit Acetoxycrotonsäuremethylester und Acetoxyvinylessigsäuremethylester als Begleitkomponenten ; entsprechendes gilt für die homologen Verbindungen.

Grundsätzlich läßt sich jedoch für die Umsetzung des AC-Esters und des AV-Esters auch ein Alkohol verwenden, der nicht der Carbalkoxygruppe dieser Ester entspricht. So läßt sich z. B. Acetoxycrotonsäuremethylester und Acetoxyvinylessigsäuremethylester ohne weiteres mit Butanol umsetzen.

Am günstigsten hat sich eine Reaktionszeit von 4 bis 6 Stunden erwiesen. In dieser Zeit findet eine weitgehende Umsetzung statt. Diese beträgt unter den angegebenen Bedingungen 65-95 %. Das Reaktionsgemisch wird nach erfolgter Umsetzung vorzugsweise zusammen mit dem bei der Reaktion von Diketen mit einem Alkohol entstehenden Rohprodukt aufgearbeitet, daher ist eine vollständige Umsetzung des AC- und des AV-Esters beim erfindungsgemäßen Verfahren nicht erforderlich und aus wirtschaftlichen Gründen sogar nicht zweckmäßig.

Die Aufarbeitung des erwähnten, bei der Umsetzung von Diketen mit einem Alkohol entstehenden Rohprodukt vereinfacht sich also durch die Kopplung mit dem erfindungsgemäßen Verfahren: Nach der Abtrennung der Hoch- und Niedrigsieder von dem Rohprodukt destilliert man nur die Hauptmenge des Acetessigsäurealkylesters ab (anstatt ihn wie bisher weitestgehend abzudestillieren) und setzt die Restmenge gemeinsam mit AC- und AV-Ester im erfindungsgemäßen Verfahren ein.

Für das erfindungsgemäße Verfahren wird vorzugsweise ein Gemisch eingesetzt, das einen möglichst hohen Anteil an AC- und AV-Ester enthält. Man gewinnt ein solches Gemisch aus dem Sumpf der Kolonne, in der die Reindestillation des Acetessigsäurealkylesters nach Abtrennung der Hoch- und Niedrigsieder durchgeführt wird. Die Konzentration an Acetessigsäurealkylester beträgt hier zwischen 20 und 80 % Gew., vorzugsweise 40 bis 50 % Gew. Doch auch bei wesentlich höheren Werten läuft die Umsetzung ohne Schwierigkeiten ab.

Als Alkoholkomponente beim erfindungsgemäßen Verfahren eignen sich grundsätzlich alle aliphatischen Alkohole, die 1 bis 5 Kohlenstoffatome enthalten. Die Kohlenstoffkette kann geradkettig oder verzweigt sein, es kann sich um primäre, sekundäre oder tertiäre Alkohole handeln; ferner sind auch ungesättigte Alkohole einsetzbar. So sind z. B. als Reaktionspartner folgende Alkohole zu verwenden: Methanol, Ethanol, Propanol-(1), Propanol-(2), Butanol-(1), Butanol-(2), 2-Methyl-propanol-(1), 2-Methylpropanol-(2), Pentanol-(1), Pentanol-(2), Pentanol-(3), 2-Methyl-butanol-(4), 2-Methyl-butanol-(3), 2-Methylbutanol-(2), 2-Methyl-butanol-(1), 2,2-Dimethyl-propanol-(1), Propen-(1)-ol-(3). Bevorzugt sind primäre Alkohole, insbesondere Methanol und Ethanol.

Besonders günstig sind die beiden folgenden Ausführungsformen des erfindungsgemäßen Verfahrens:

a) Aus dem Rohprodukt, das bei der kontinuierlichen Herstellung von Acetessigsäuremethylester aus Diketen und Methanol entsteht, werden Hoch- und Niedrigsieder abgetrennt und anschließend die Hauptmenge des Acetessigsäuremethylesters. Als Sumpfprodukt erhält man 3-Acetoxycrotonsäuremethylester, 3-Acetoxyvinylessigsäuremethylester und den Rest des Acetessigsäuremethylesters. Dieses Sumpfprodukt wird erfindungsgemäß mit Methanol zu Acetessigsäuremethylester umgesetzt. Das hierbei erhaltene Produkt wird mit dem oben erwähnten Rohprodukt (aus der kontinuierlichen Umsetzung von Diketen und Methanol zu Acetessigsäuremethylester) vereinigt und gemeinsam mit diesem zu reinem Acetessigsäuremethylester aufgearbeitet.

b) Aus dem Rohprodukt, das bei der kontinuierlichen Herstellung von Acetessigsäureethylester aus Diketen und Ethanol entsteht, werden Hoch- und Niedrigsieder abgetrennt und anschließend die Hauptmenge des Acetessigsäureethylesters. Als Sumpfprodukt erhält man 3-Acetoxycrotonsäureethylester, 3-Acetoxyvinylessigsäureethylester und den Rest des Acetessigsäureethylesters. Dieses Sumpfprodukt wird erfindungsgemäß mit Ethanol zu Acetessigsäureethylester umgesetzt. Das hierbei erhaltene Produkt wird mit dem oben erwähnten Rohprodukt (aus der kontinuierlichen Umsetzung von Diketen und Ethanol zu Acetessigsäureethylester) vereinigt und gemeinsam mit diesem zu reinem Acetessigsäureethylester aufgearbeitet.

Die folgenden Beispiele erläutern das erfindungsgemäße Verfahren:

Beispiel 1

Es wurden 50 g einer Mischung eingesetzt, die folgende Zusammensetzung hatte: 53,0 Gew.% Methanol, 45,9 Gew.% 3-Acetoxycrotonsäuremethylester (cis- und trans- Form) und 3-Acetoxyvinylessigsäuremethylester, 0,6 Gew.% Acetessigsäuremethylester, sowie 0,5 Gew.% sonstige Mittel- und Hochsieder. Diese Mischung wurde in einem Autoklaven 6 Stunden auf 170 °C erhitzt, wobei sich am Ende der Reaktion ein Druck von 11,3 bar einstellte. Nach dem Abkühlen erfolgte erneut eine Analyse der Reaktionsmischung, die folgende Zusammensetzung ergab: 67,2 Gew.% Niedrigsieder, (im wesentlichen Methylacetat und Methanol) 31,4 Gew.% Acetessigsäuremethylester, 1,4 Gew.% Acetoxycrotonsäuremethyl und Acetoxyvinylessigsäuremethylester, 0,2 Gew.% sonstige Mittel- und Hochsieder.

Beispiel 2

Es wurden 50 g einer Mischung eingesetzt, die folgende Zusammensetzung hatte: 53,6 Gew.% Methanol, 25,0 Gew.% Acetessigsäuremethylester und 21,4 Gew.% 3-Acetoxycrotonsäuremethylester (cis und trans) und 3-Acetoxyvinylessigsäuremethylester. Diese Mischung wurde in einem Autoklaven 6 Stunden auf 170 °C erhitzt, wobei sich am Ende der Reaktion ein Druck von 10,8 bar einstellte. Nach dem Abkühlen hatte eine Probe der Reaktionsmischung folgende Zusammensetzung : 57,9 Gew.% Niedrigsieder, (im wesentlichen Methylacetat und Methanol) 35,7 Gew.% Acetessigsäuremethylester, 6,1 Gew.% 3-Acetoxycrotonsäuremethylester und 3-Acetoxyvinylessigsäuremethylester, 0,3 Gew.% sonstige Mittel- und Hochsieder.

Beispiel 3

Es wurden 50 g einer Mischung eingesetzt, die folgende Zusammensetzung hatte : 51,3 Gew.% Ethanol, 0,9 Gew.% Acetessigsäureethylester, 47,5 Gew.% 3-Acetoxycrotonsäureethylester (cis und trans) und 3-Acetoxyvinylessigsäureethylester, 0,3 Gew.% sonstige Mittel- und Hochsieder. Diese Mischung wurde in einem Autoklaven 6 Stunden auf 170 °C erhitzt, wobei sich am Ende der Reaktion ein Druck von 10,1 bar einstellte. Nach dem Abkühlen hatte die Reaktionsmischung folgende Zusammensetzung : 67,0 Gew.% Niedrigsieder (im wesentlichen Ethylacetat und Ethanol), 30,5 Gew.% Acetessigsäureethylester, 1,9 Gew.% 3-Acetoxycrotonsäureethylester und 3-Acetoxyvinylessigsäureethylester, 0,6 Gew.% sonstige Mittel- und Hochsieder.

Beispiel 4

Es wurden 100 g einer Mischung eingesetzt, die folgende Zusammensetzung hatte : 44,0 Gew.% Ethanol, 25,7 Gew.% Acetessigsäureethylester, 30,1 Gew.% 3-Acetoxycrotonsäureethylester (cis und trans) und 3-Acetoxyvinylessigsäureethylester, 0,2 Gew.% sonstige Mittel- und Hochsieder. Diese Mischung wurde in einem Autoklaven 6 Stunden auf 170 °C erhitzt, wobei sich am Ende der Reaktion ein Druck von 9,7 bar einstellt. Nach dem Abkühlen hatte eine Probe der Reaktionsmischung folgende Zusammensetzung : 53,1 Gew.% Niedrigsieder, (im wesentlichen Ethylacetat und Ethanol), 35,4 Gew.% Acetessigsäureethylester, 10,6 Gew.% 3-Acetoxycrotonsäureethylester und 3-Acetoxyvinylessigsäureethylester, 0,9 Gew.% sonstige Mittel- und Hochsieder.

**Ansprüche**

1. Verfahren zur Herstellung von Acetessigsäurealkylestern, dadurch gekennzeichnet, daß man 3-Acetoxycrotonsäurealkylester oder 3-Acetoxyvinylessigsäurealkylester oder deren Gemisch bei einer Temperatur von 50 bis 250 °C mit einem aliphatischen Alkohol, der 1 bis 5 Kohlenstoffatome enthält, in der Flüssigphase umsetzt, wobei der Alkohol in mindestens äquimolarer Menge eingesetzt wird.

2. Verfahren nach Anspruch 1, dadurch gekennzeichnet, daß die Alkylgruppen der 3-Acetoxycrotonsäurealkylester oder der 3-Acetoxyvinylessigsäurealkylester gesättigt oder ungesättigt, geradkettig oder verzweigt sind und 1 bis 5 Kohlenstoffatome enthalten.

3. Verfahren nach Anspruch 1 oder 2, dadurch gekennzeichnet, daß der Alkohol dieselbe Alkylgruppe enthält wie der 3-Acetoxycrotonsäurealkylester oder der 3-Acetoxyvinylessigsäurealkylester.

4. Verfahren nach Anspruch 1 oder 3, dadurch gekennzeichnet, daß man einen primären aliphatischen Alkohol einsetzt.

5. Verfahren nach Anspruch 1 oder 3, dadurch gekennzeichnet, daß man als aliphatischen Alkohol Methanol oder Ethanol einsetzt.

6. Verfahren nach einem der Ansprüche 1 bis 5, dadurch gekennzeichnet, daß man 3-Acetoxycrotonsäurealkylester und 3-Acetoxyvinylessigsäurealkylester im Gemisch mit Acetessigsäurealkylester einsetzt, wobei alle drei Ester die gleiche Alkylgruppe haben und wobei der Anteil des Acetessigsäurealkylesters im Gemisch 40-50 Gew.% beträgt.

7. Verfahren nach einem der Ansprüche 1 bis 6, dadurch gekennzeichnet, daß man die Umsetzung bei 100 bis 200 °C durchführt.

**Claims**

1. Process for the preparation of acetoacetic acid alkyl esters characterized in that 3-acetoxycrotonic acid alkyl esters or 3-acetcxyvinylacetic acid alkyl esters or a mixture thereof are reacted in the liquid phase at a temperature of from 50 to 250 °C with an aliphatic alcohol which contains 1 to 5 carbon atoms, the alcohol being employed in at least equimolar quantity.

2. Process as claimed in claim 1, characterized in that the alkyl groups of the 3-acetoxycrotonic acid alkyl esters or the 3-acetoxyvinylacetic acid alkyl esters are saturated or unsaturated, straight-chain or branched and contain 1 to 5 carbon atoms.

3. Process as claimed in claim 1 or 2, characterized in that the alcohol contains the same alkyl group as the 3-acetoxycrotonic acid alkyl ester or the 3-acetoxyvinylacetic acid alkyl ester.

4. Process as claimed in claim 1 or 3, characterized in that a primary aliphatic alcohol is employed.

5. Process as claimed in claim 1 or 3, in which methanol or ethanol is employed as the aliphatic alcohol.

6. Process as claimed in anyone of claims 1 to 5, characterized in that 3-acetoxycrotonic acid alkyl ester and 3-acetoxyvinylacetic acid alkyl ester are employed in mixtures with acetoacetic acid alkyl esters, all three esters having the same alkyl group and the portion of the acetoacetic acid alkyl ester in the mixture being 40 to 50 % by weight.

7. Process as claimed in one of claims 1 to 6, characterized in that the reaction is carried out at 100 to 200 °C.

**Revendications**

1. Procédé pour préparer des acétylacétates d'alkyles, procédé caractérisé en ce qu'on fait réagir un acétoxy-3 crotonate d'alkyle ou un acétoxy-3 vinylacétate d'alkyle, ou un mélange de tels esters, en phase liquide, à une température de 50 à 250 °C, avec un alcool aliphatique contenant de 1 à 5 atomes de carbone, l'alcool étant utilisé en une quantité au moins égale à la quantité molaire.

2. Procédé selon la revendication 1 caractérisé en ce que les radicaux alkyles des acétoxy-3 crotonate d'alkyles ou des acétoxy-3 vinylacétate d'alkyles sont saturés ou insaturés, linéaires ou ramifiés, et contiennent de 1 à 5 atomes de carbone.

3. Procédé selon l'une des revendications 1 et 2, caractérisé en ce que l'alcool contient le même radical alkyle que l'acétoxy-3 crotonate d'alkyle ou l'acétoxy-3 vinylacétate d'alkyle.

4. Procédé selon l'une des revendications 1 et 3, procédé caractérisé en ce qu'on utilise un alcool aliphatique primaire.

5. Procédé selon l'une des revendications 1 et 3, caractérisé en ce qu'on utilise, comme alcool aliphatique, le méthanol ou l'éthanol.

6. Procédé selon l'une quelconque des revendications 1 à 5, caractérisé en ce qu'on utilise l'acétoxy-3 crotonate d'alkyle et l'acétoxy-3 vinylacétate d'alkyle en mélange avec l'acétylacétate d'alkyle, les trois esters contenant le même radical alkyle et la teneur du mélange en l'acétylacétate d'alkyle étant comprise entre 40 et 50 % en poids.

7. Procédé selon l'une quelconque des revendications 1 à 6, caractérisé en ce que la réaction est effectuée à une température de 100 à 200 °C.